# EUROPEAN PATENT SPECIFICATION

(11) **EP 3 510 332 B1**
(45) Date of publication and mention of the grant of the patent: **26.08.2020**
(21) Application number: 17772174.3
(22) Date of filing: 08.09.2017
(51) Int. Cl.: F26B 5/06

(54) **REPEATED STERILE USE OF A GAUGE IN A STEAM STERILIZABLE FREEZE-DRYING SYSTEM**
WIEDERHOLTE STERILE VERWENDUNG EINES MESSGERÄTS IN EINEM DAMPFSTERILISIERBAREN GEFRIERTROCKNUNGSSYSTEM
UTILISATION STÉRILE RÉPÉTÉE D'UNE JAUGE DANS UN SYSTÈME DE LYOPHILISATION STÉRILISABLE À LA VAPEUR

(30) Priority: 09.09.2016 US 201615260569
(43) Date of publication of application: 17.07.2019
(73) Proprietor: SP Industries, Inc., Warminster, PA 18974 (US)
(72) Inventor: DERN, Charles D., Doylestown, Pennsylvania 18902 (US)
(74) Representative: IP Sextant s.r.l.
(86) International application number: PCT/US2017/050749
(87) International publication number: WO 2018/049208

(56) References cited:
- CN-A- 101 614 469
- CN-A- 105 148 301
- CN-U- 205 108 463
- CN-Y- 2 888 374
- DE-A1-102009 008 970

## Description

### FIELD OF THE INVENTION

The present disclosure relates to field of freeze-drying systems, and more particularly, to repeated sterile use of gauges or other heat sensitive instruments in steam sterilizable freeze-drying systems.

### BACKGROUND OF THE INVENTION

Freeze-drying (e.g., lyophilization, cryodesiccation) is a process to remove water and/or other solvents from products. Freeze-drying has many applications such as preserving a perishable material, making a material more convenient for transport, making of ceramics, producing a product that has a short reconstitution time with acceptable potency levels, and so forth. Freeze-drying can be used on many materials, including food, pharmaceuticals, and so forth. DE102009008970 A1 discloses a freeze-drying system comprising a freeze-drying chamber wherein products can be arranged over supports, CN 105148301 A further teaches a device for storing medicines comprising a refrigerating air dryer, a sterilization tank and a drying chamber.

### BRIEF DESCRIPTION OF THE DRAWINGS

The present disclosure is illustrated by way of example, and not by way of limitation, in the figures of the accompanying drawings.
FIG. 1 is a schematic view of a freeze-drying system in accordance with one embodiment.
FIG. 2A is a schematic view of a Pirani gauge of the freeze-drying system in accordance with one embodiment.
FIG. 2B is a schematic view of a thermocouple gauge of the freeze-drying system in accordance with one embodiment.
FIG.3 is a schematic view of a capacitance manometer of the freeze-drying system in accordance with one embodiment.
FIG. 4 is a graph of pressure measurements received from a gauge and a capacitance of the freeze-drying system in accordance with one embodiment.
FIG. 5 is a schematic view of a freeze-drying system in accordance with another embodiment.
**FIG. 6** illustrates a flow diagram of a method for controlling valves during a steam sterilization (SIP) cycle of a freeze-drying system, in accordance with one embodiment.
**FIG. 7** illustrates a flow diagram of a method for determining an endpoint of a primary drying stage of a freeze-drying system, in accordance with one embodiment.

### DETAILED DESCRIPTION OF THE INVENTION

Freeze-drying includes at least a freezing stage, a primary drying (e.g., sublimation) stage, and a secondary drying (e.g., desorption) stage. During the freezing stage, a product is frozen and ice crystals are formed in the product. During the primary drying stage, unbound water is removed from the product via sublimation of the free ice crystals by an increase in temperature. During the secondary drying stage, the temperature is raised higher to remove bound water molecules from the product.

Freeze-drying can be an expensive and time-consuming process. The primary drying stage may be slow (in some instances taking multiple days to complete) and may take longer to complete than any of the other stages. Also, if the secondary drying stage is started before all of the frozen water is removed from the product, the product may be damaged. Therefore, determination of the endpoint of the primary drying stage is highly valuable as it allows a shorter primary drying stage, helps avoid damage to the product, helps maximize throughput of product through the freeze-drying system, and increases profit.

The endpoint of primary drying can be determined by taking pressure measurements. Pressure measurements can be provided by a gauge, such as a Pirani gauge, a thermocouple gauge, or a manometer.

Freeze-drying systems may be sterilized by passing steam through the freeze-drying system in a steam sterilization (SIP) cycle. A gauge can be damaged by contact with steam and may need to be replaced each time the gauge is contacted by steam. Frequent replacement of the gauges can be very costly. There is a need for an assembly, a method, and a system to allow repeated sterile use of a gauge in a steam sterilizable freeze-drying system.

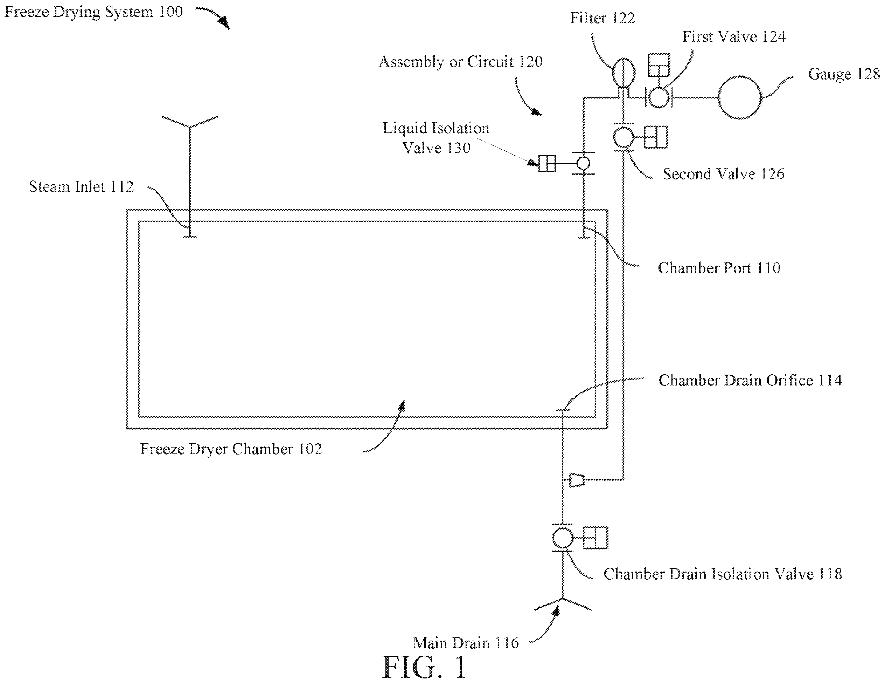

**FIG. 1** is a schematic view of a freeze-drying system 100 in accordance with one embodiment. A freeze-drying system 100 may include a freeze dryer chamber 102 that includes one or more orifices in fluid communication with the freeze dryer chamber 102. The one or more orifices may include a first orifice or chamber port 110, a second orifice or steam inlet 112, and a third orifice or chamber drain orifice 114. Steam may enter the freeze dryer chamber 102 via the steam inlet 112. The steam inlet 112 is disposed upstream from the chamber port 110 and the chamber drain orifice 114. The chamber drain orifice 114 is disposed upstream from a main drain 116. A chamber drain isolation valve 118 may be disposed downstream from the chamber drain orifice 114 and upstream from the main drain 116.

In one embodiment, the freeze dryer chamber 102 is a manifold with attached flasks. In another embodiment, the freeze dryer chamber 102 is a chamber with a system of shelves on which to place product. In another embodiment, the freeze dryer chamber is a combination of a chamber with shelves and a manifold with attached flasks.

The freeze-drying system 100 may include an assembly or circuit 120 coupled to the freeze dryer chamber 102 via the chamber port 110. The circuit 120 may include a filter 122 disposed downstream from the freeze dryer chamber 102, a first valve 124 disposed downstream from the filter 122, a second valve 126 disposed downstream from the filter 122, and a gauge 128 disposed downstream from the first valve 124. The circuit 120 may include a liquid isolation valve 130 disposed downstream from the freeze dryer chamber 102 and upstream from the filter 122. In one embodiment, the assembly or circuit 120 is retrofitted to an existing freeze-drying system. In another embodiment, the assembly or circuit 120 is coupled to new freeze-drying system.

The freeze-drying system 100 may perform freeze-drying of a product. Freeze-drying may include stages (e.g., freezing stage, primary drying stage, secondary stage, and so forth). In one embodiment, the freeze-drying system 100 performs a steam sterilization (SIP) cycle. In another embodiment, the freeze drying system performs a clean-in-place (CIP) cycle. A freeze-drying system 100 may perform one or more of a SIP cycle and/or a CIP cycle after each use of the freeze-drying system 100 to ensure that a product is not contaminated by material previously lyophilized in the freeze-drying system 100. The SIP cycle may include sanitizing the freeze dryer chamber 102 with steam. The CIP cycle may include rinsing the inside of the freeze dryer chamber 102 with a media (e.g., a liquid, water, deionized water, Water for Injection (WFI), detergent, caustic material, acid, and so forth). The liquid isolation valve 130 may isolate the filter 122 and gauge 128 from the media of the CIP cycle.

The filter 122 according to the present invention includes a steam sterilizable filter. The filter 122 may have a nominal pore size of 0.22 microns or less. In one embodiment, the gauge 128 may not be sterilized (e.g., is heat sensitive, may be damaged or lose efficiency after one or more SIP cycles). During freeze-drying cycles, the "non-sterile" gauge 128 is open to the freeze-drying process via the sterilized filter 122. Although the gauge 128 may not be sterilized, since the gauge 128 is located downstream from the sterilized filter 122, the sterilized filter 122 may maintain the sterile envelope required by Good Manufacturing Practices (GMP). In another embodiment, the filter 122 may not be contacted by the media of the CIP cycle (e.g., may be damaged or lose efficiency after one or more contacts with the media used in the CIP cycle). During CIP cycles, the liquid isolation valve 130 may be in a closed position. During freeze-drying cycles, the liquid isolation valve 130 may be open to the freeze drying process.

During a SIP cycle, the first valve 124 is in a closed position to isolate the gauge 128 from the steam. The first valve 124 in the closed position according to the present invention protects the gauge 128 from damage caused by contact with the steam. In one embodiment, the first valve 124 is in an open position except for during the SIP cycles. In another embodiment, the first valve 124 is in the open position during at least the primary drying stage. In another embodiment, the first valve 124 is in the open position during all operations of the freeze-drying system 100 except for the SIP cycles. In another embodiment, the gauge 128 is isolated from the freeze dryer chamber 102 by the first valve 124 during the SIP cycle.

During the SIP cycle, the second valve 126 is in an open position. In one embodiment, the second valve 126 is in the closed position except for during the SIP cycles. In another embodiment, the second valve 126 is in the closed position during all operations of the freeze-drying system 100 except for the SIP cycles. In another embodiment, the second valve 126 is in an open position when the first valve 124 is in a closed position. In another embodiment, the second valve 126 is in a closed position when the first valve 124 is in an open position.

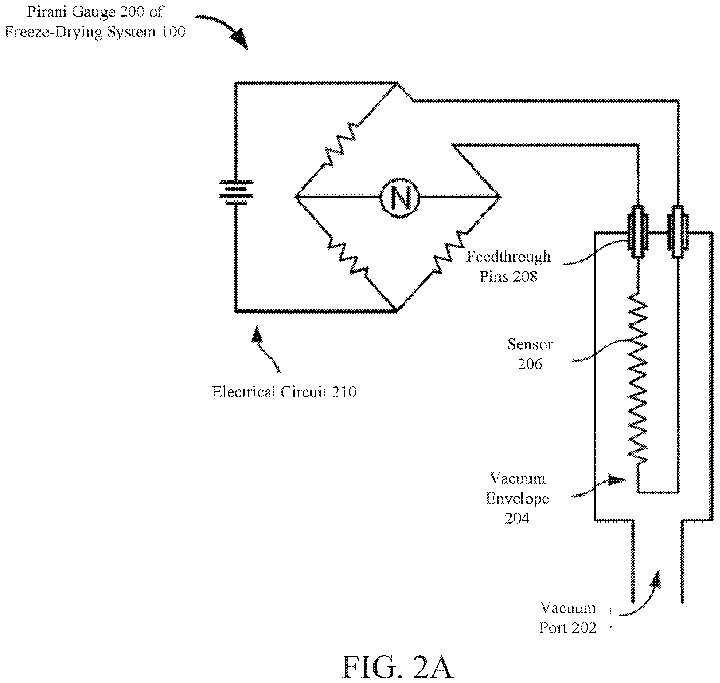

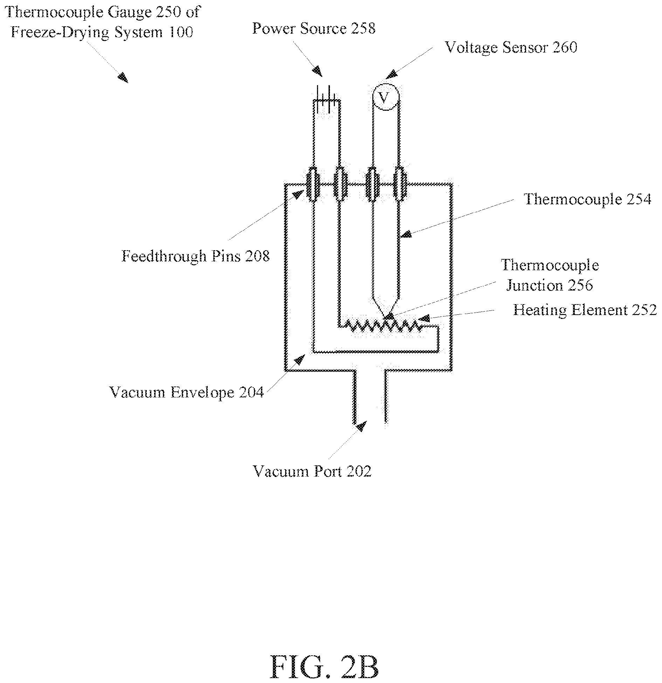

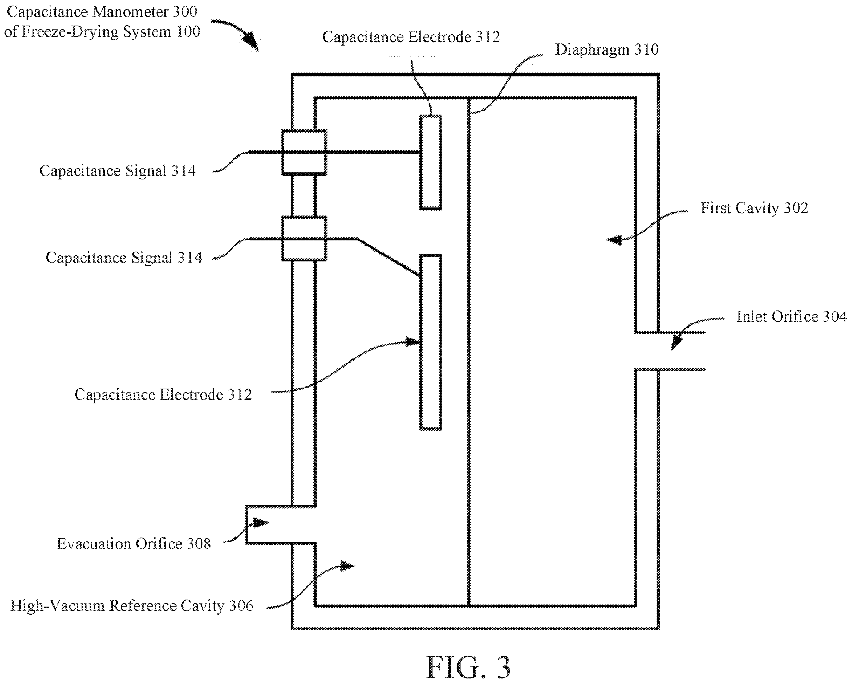

The gauge 128 is disposed downstream from the first valve 124 and downstream from the liquid isolation valve 130. The gauge 128 may determine a plurality of measurements (e.g., pressure measurements) of the freeze-drying system 100. The gauge may determine the plurality of measurements when the freeze-drying system 100 is not in a SIP cycle. The gauge 128 may determine the plurality of measurements during repeated sterile uses of the gauge 128 (e.g., a first plurality of measurements before a SIP cycle and a second plurality of measurements after the SIP cycle). In one embodiment, the gauge 128 takes pressure measurements. In another embodiment, the gauge 128 is a heat-sensitive instrument (e.g., is susceptible to steam, is damaged if in contact with steam) that is a gauge. In another embodiment, the gauge 128 is a heat-sensitive instrument that is not a gauge (e.g., any instrument or part that is susceptible to steam and is relevant to the freeze-drying system 100). In another embodiment, the gauge 128 is a vacuum gauge. In another embodiment, the gauge 128 is a manometer. In another embodiment, the gauge 128 is a thermocouple (e.g., a thermocouple gauge 250, see FIG. 2B). In another embodiment, the gauge 128 is a Pirani gauge 200 (see FIG. 2A). In another embodiment, the gauge 128 is two or more instruments or parts (e.g., a capacitance manometer 300 (see FIG. 3) and a Pirani gauge 200) and at least one of the instruments or parts is heat-sensitive (e.g., the Pirani gauge 200).

In one embodiment, the gauge 128 may be disposed downstream from the first valve 124 and the filter 122 in the freeze-drying system 100 in a similar configuration as how a gas supply (e.g., a bottle of nitrogen) and a metering valve are disposed downstream from a valve and a filter in a gas bleed subsystem of a freeze-drying system. In a gas bleed subsystem of a freeze-drying system, a steady stream of gas (e.g., nitrogen) may be supplied from the gas supply and the meter valve may control the stream of gas to maintain a vacuum level control. The metering valve may be a delicate valve that cannot be sterilized without damaging the metering valve. The freeze-drying system containing a gas bleed subsystem may be sterile up through the filter to the first valve. The freeze-drying system 100 may also be sterile up through the filter 122 to the first valve 124. The gauge 128 of the freeze-drying system 100 may be a type of instrument that cannot be sterilized without damaging the gauge 128. In one embodiment, the freeze-drying system 100 includes a gas bleed subsystem.

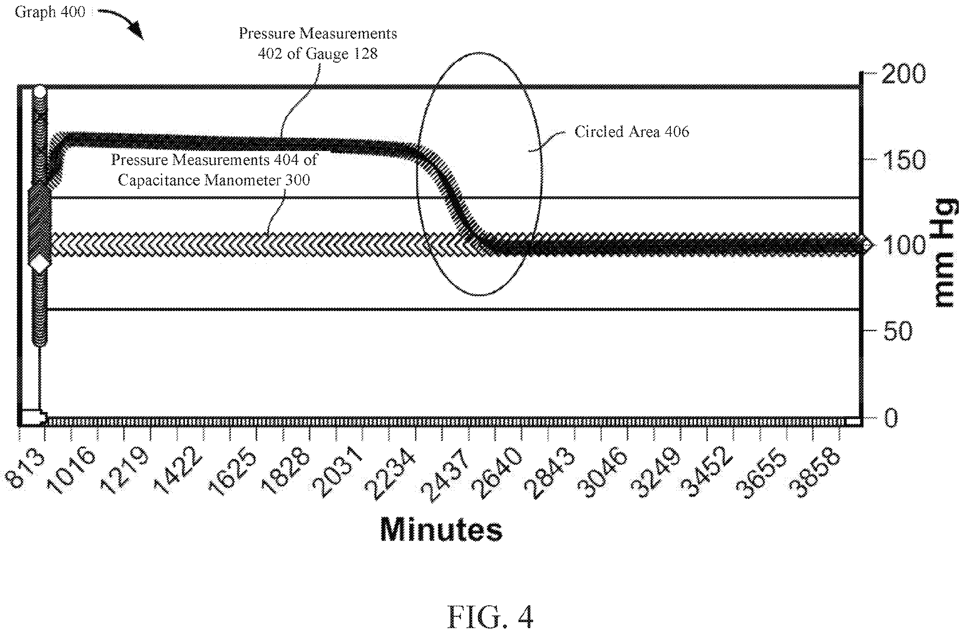

The freeze-drying system 100 may include a first instrument (e.g., capacitance manometer 300) that gives accurate pressure measurements regardless the amount of water vapor in the freeze dryer chamber 102 and a second instrument (e.g., gauge 128, Pirani gauge 200, thermocouple 250) that gives erroneous pressure measurements given the amount of water vapor in the freeze dryer chamber 102. The freeze-drying system 100 may also include a processing device to determine, in view of at least one of the plurality of measurements taken by the gauge 128, an endpoint of a primary drying stage of the freeze-drying system 100 (e.g., by comparing pressure measurements 404 of the capacitance manometer 300 to pressure measurements 402 of gauge 128, see FIG. 4). In one embodiment, the processing device is coupled to the freeze-drying system 100. In another embodiment, the processing device is integral to the freeze-drying system 100.

**FIG. 2A** is a schematic view of a Pirani gauge 200 of the freeze-drying system 100 in accordance with one embodiment. In one embodiment, the gauge 128 may include a Pirani gauge 200. The Pirani gauge 200 may include a vacuum port 202 coupled to the freeze dryer chamber 102. The vacuum port 202 may be disposed downstream from the first valve 124 and downstream from the filter 122. The Pirani gauge 200 may include a vacuum envelope 204 downstream from the vacuum port. A sensor 206 may be disposed within the vacuum envelope 204. The sensor 206 may be coupled via feedthrough pins 208 to an electrical circuit 210 (e.g., a Wheatstone-bridge) to measure the electrical resistance of the sensor 206.

The sensor 206 may be heated metal filament suspended in the gas of the vacuum envelope 204. The sensor 206 may lose heat to the gas molecules in the vacuum envelope 204 as the gas molecules collide with the sensor 206. If the gas pressure is reduced, the number of molecules present will fall proportionally and the sensor 206 will rise in temperature due to the reduced cooling effect. The electrical resistance of the sensor 206 varies with the temperature of the sensor 206. The electrical resistance can be used to calculate the pressure of the gas in the vacuum envelope 204. The Pirani gauge 200 may be calibrated in a type of gas (e.g., pure nitrogen) and the Pirani gauge 200 may give erroneously high pressure measurements when there is water vapor present in the freeze dryer chamber 102.

The Pirani gauge 200 may become damaged if contacted with steam. The sensor 206 may become damaged and stop functioning after one contact with steam (e.g., one SIP cycle). During SIP cycles, the first valve 124 may be in a closed position to prevent steam from contacting Pirani gauge 200 and to prevent damage of Pirani gauge 200 and sensor 206.

**FIG. 2B** is a schematic view of a thermocouple gauge 250 of the freeze-drying system 100 in accordance with one embodiment. In one embodiment, the gauge 128 may include the thermocouple gauge 250. The thermocouple gauge 250 may include a vacuum port 202 coupled to the freeze dryer chamber 102. The vacuum port 202 may be disposed downstream from the first valve 124 and downstream from the filter 122. The thermocouple gauge 250 may include a vacuum envelope 204 downstream from the vacuum port. A heating element 252 and a thermocouple 254 may be disposed within the vacuum envelope 204. The heating element 252 may be a heating wire and the thermocouple 254 may include two different metals joined at a thermocouple junction 256. The thermocouple junction 256 may be attached to the center of the heating element 252. The heating element 252 may be coupled via feedthrough pins 208 to a power source 258. The thermocouple 254 may be coupled via feedthrough pins 208 to a voltage sensor 260 to determine the temperature at the thermocouple junction 256.

The heating element 252 may be energized by the power source 258 to a constant current and heat generated by the heating element 252 may be removed at a rate that depends on the thermal conductivity of the gas in the vacuum envelope 204. The temperature detected at the thermocouple junction 256 depends on the thermal conductivity of the surrounding gas. The thermal conductivity of the gas may depend on the pressure of the gas and the type of gas (e.g., pure nitrogen, amount of water vapor). If the gas pressure is reduced, the number of molecules present will fall proportionally and the thermocouple 254 will measure a rise in temperature due to the reduced cooling effect. The temperature measured by the voltage sensor 260 can be used to calculate the pressure of the gas in the vacuum envelope 204. The thermocouple gauge 250 may be calibrated in a type of gas (e.g., pure nitrogen) and the thermocouple gauge 250 may give erroneously high pressure measurements when there is water vapor present in the freeze dryer chamber 102.

The thermocouple gauge 250 may become damaged if contacted with steam. One or more components of the thermocouple gauge 250 (e.g., heating element 252, thermocouple 254, thermocouple junction 256, and so forth) may become damaged and stop functioning after one or more SIP cycles. During SIP cycles, the first valve 124 may be in a closed position to prevent steam from contacting thermocouple gauge 250 and to prevent damage of thermocouple gauge 250, heating element 252, thermocouple 254, and thermocouple junction 256.

**FIG. 3** is a schematic view of a capacitance manometer 300 of the freeze-drying system 100 in accordance with another embodiment. The capacitance manometer 300 may include a first cavity 302 that has an inlet orifice 304 coupled to the freeze dryer chamber 102. The first cavity may be disposed downstream from the first valve 124 and downstream from the filter 122. The capacitance manometer 300 may include a high-vacuum reference cavity 306 that is evacuated and sealed at an evacuation orifice 308 of the high-vacuum reference cavity. The capacitance manometer 300 may include a diaphragm 310 (e.g., a tensioned Inconel® diaphragm) separating the first cavity 302 from the high-vacuum reference cavity 306. The capacitance manometer 300 may include capacitance electrodes 312 located within the high-vacuum reference cavity 306 and coupled to capacitance signals 314.

As the pressure in the freeze dryer chamber 102 changes, there will be a change in pressure on the diaphragm 310 causing the diaphragm 310 to move. There will be a direct correspondence between pressure input and voltage output as the distance between the diaphragm 310 and the capacitance electrodes 312 changes. The signal conditioning electronics of a capacitance manometer 300 may provide an output varying from 0 to 10 volts with a linear relationship between output voltage and pressure. Pressure measurements of the capacitance manometer 300 may be independent of the composition of the vapor or gas mixture in the vacuum space, therefore a capacitance manometer 300 may give true vacuum readings that are not erroneously influenced by water vapor.

**FIG. 4** is a graph 400 of pressure measurements 402, 404 received from a gauge 128 and a capacitance manometer 300 of a freeze-drying system 100 in accordance with one embodiment. In one embodiment, gauge 128 includes a Pirani gauge 200. In another embodiment, gauge 128 includes a thermocouple gauge 250. In one embodiment, both the gauge 128 and the capacitance manometer 300 are disposed downstream from filter 122 and first valve 124. In another embodiment, at least the gauge 128 is located downstream from the filter 122 and the first valve 124.

The gauge 128 provides erroneous (e.g., artificially high) pressure measurements 402 proportional to the amount of water vapor present in the freeze dryer chamber 102. A capacitance manometer 300 provides pressure measurements 404 that are not erroneously influenced by water vapor in the freeze dryer chamber 102. The primary drying stage sublimates frozen water to water vapor, thereby producing water vapor in the freeze dryer chamber 102. At the end of the primary drying stage, there is little or no water vapor in the freeze dryer chamber 102.

Graph 400 plots the pressure measurements 402 from gauge 128 and the pressure measurements 404 from capacitance manometer 300 over time. Initially, there may be a difference between the pressure measurements 402 and the pressure measurements 404 of about 50 mm Hg (millimeter of mercury). As the amount of water vapor in the freeze dryer chamber 102 begins to recede, the pressure measurements 402 will decrease concomitantly with the decrease in water vapor until there is little or no more water vapor leaving the product in the freeze dryer chamber 102. The circled area 406 shows where the pressure measurements 402 and the pressure measurements 404 converge and the circled area 406 marks the endpoint of the primary drying stage.

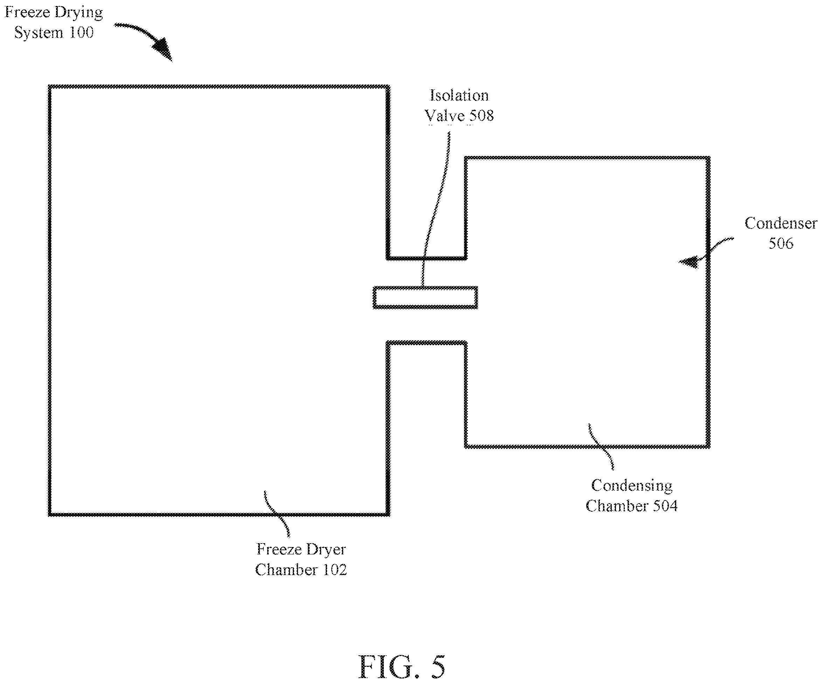

**FIG. 5** is a schematic view of a freeze-drying system 100 in accordance with another embodiment. The freeze-drying system 100 includes a freeze dryer chamber 102. The freeze-drying system 100 may include a condensing chamber 504. In one embodiment, an ice condenser 506 is located inside the condensing chamber 504. In another embodiment, the ice condenser 506 is located inside chamber 102. An isolation valve 508 may separate the condensing chamber 504 from the freeze dryer chamber 102. The isolation valve 508 may be a butterfly-type valve that sits in a spool (e.g., a throat, a vapor port, and so forth) located between the freeze dryer chamber 102 and the condensing chamber 504. When the isolation valve 508 is in an open position, vapor may flow from the freeze dryer chamber 102 to the condenser 506 in the condensing chamber 504. When the isolation valve is in a closed position, vapor flow is prevented from flowing from the freeze dryer chamber 102 to the condensing chamber 504. By closing the isolation valve 508 for a short period of time, the vapor liberated from the product remains in the freeze dryer chamber 102 and a gauge (e.g., gauge 128 (e.g., Pirani gauge 200, thermocouple gauge 250), capacitance manometer 300, two or more gauges, and so forth) provides a pressure rise measurement of the pressure in the freeze dryer chamber 102. The isolation valve remains in the open position the majority of the time and is closed for a short period at regular intervals. As the primary drying stage moves towards completion, the pressure rise in the freeze dryer chamber 102 when the isolation valve 508 is closed will decrease. When the gauge provides a pressure rise measurement indicating the pressure rise is below a threshold (e.g., approximately 5 mTorr (millitorr)), there is so little vapor being generated that the freeze-drying system 100 is at the endpoint of the primary drying stage.

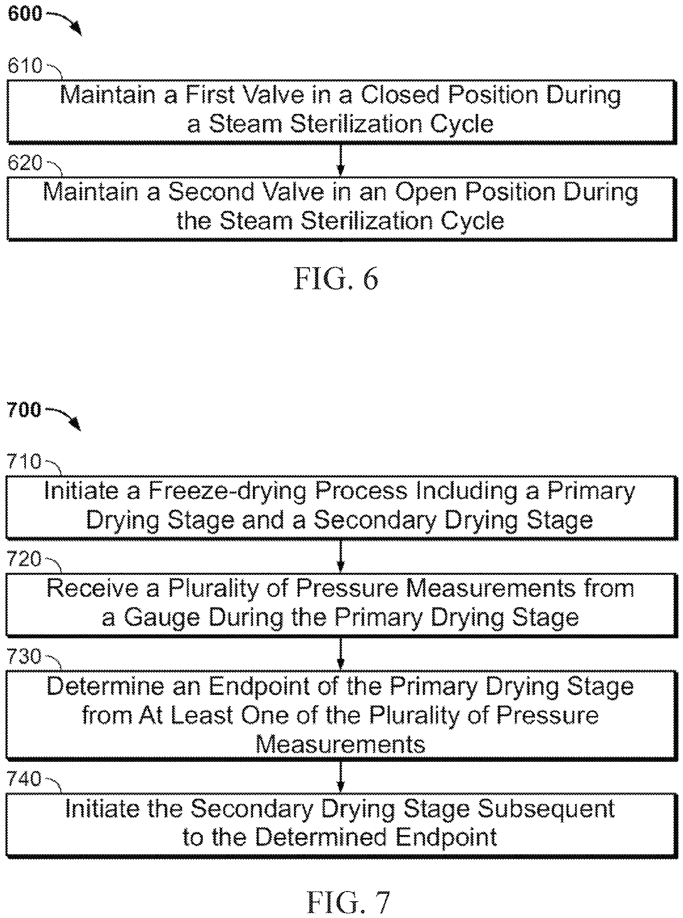

**FIG. 6** illustrates a flow diagram of a method 600 for controlling valves (e.g., first valve 124, second valve 126) during a SIP cycle of a freeze-drying system 100, in accordance with one embodiment.

Method 600 is described in relation to the freeze-drying system 100. However, it should be understood that method 600 may also be used by other systems. The method 600 may be performed by processing logic that comprises hardware (e.g., circuitry, dedicated logic, programmable logic, microcode, etc.), software (e.g., instructions run on a processing device to perform hardware simulation), or a combination thereof.

At block 610, processing logic maintains a first valve 124 in a closed position during a SIP cycle. The first valve 124 is associated with an assembly or circuit 120 coupled to a freeze dryer chamber 102. The circuit 120 includes a filter 122 disposed downstream from the freeze dryer chamber 102 and upstream from the first valve 124, a second valve 126 disposed downstream from the filter 122, and a gauge 128 disposed downstream from the first valve 124. In one embodiment, the processing logic may maintain the first valve 124 in the open position except for during the SIP cycle. In another embodiment, the processing logic maintains the first valve 124 in the open position during the primary drying stage. In another embodiment, the gauge 128 is isolated from the freeze dryer chamber 102 by the first valve 124 during the SIP cycle.

At block 620, processing logic maintains the second valve 126 in an open position during the SIP cycle. In one embodiment, the processing logic maintains the liquid isolation valve 130 in an open position during the SIP cycle. In another embodiment, the processing logic maintains the second valve 126 in the closed position except for during the SIP cycle. During the SIP cycle, steam enters the filter 122 from the freeze dryer chamber 102, the steam flows through the filter 122, and exits via the second valve 126 and continues according to the present invention to a connection point to the main drain 116, upstream of the chamber drain isolation valve 118.

It should be noted that the above described operations are just one method for using a freeze-drying system 100 and that, in alternative embodiments, certain ones of the operations of FIG. 6 may be optional or take a simpler form.

**FIG. 7** illustrates a flow diagram of a method for determining an endpoint of a primary drying stage of a freeze-drying system 100, in accordance with one embodiment.

Method 700 is described in relation to the freeze-drying system 100. However, it should be understood that method 700 may also be used by other systems. The method 700 may be performed by processing logic that comprises hardware (e.g., circuitry, dedicated logic, programmable logic, microcode, etc.), software (e.g., instructions run on a processing device to perform hardware simulation), or a combination thereof.

At block 710, processing logic initiates a freeze-drying process including a primary drying stage and a secondary drying stage. The freeze-drying process may include one or more of a pretreatment or formulation stage, a loading or container (e.g., bulk, flask, vials, and so forth) stage, a freezing (e.g., thermal treatment) stage at atmospheric pressure, a primary drying (sublimation) stage under vacuum, a secondary drying (desorption) stage under vacuum, a backfill and stoppering (for product in vials) stage under partial vacuum, and removal of dried product from the freeze dryer chamber 102.

During the freezing stage, the product is frozen in view of properties of the product. Ice crystals may be formed in the product.

During the primary drying stage, unbound water is removed from the product via sublimation of free ice crystals. The primary drying (sublimation) stage is a slow process (e.g., multiple days) conducted at cooler temperatures, safely below the product's critical temperature (e.g., the maximum temperature that the product can withstand without melting or collapsing) to avoid collapse. Heat energy drives the phase change of the ice crystals from solid to gas by conduction, convection, and radiation. A condenser 506 may be cooled to remove the water vapor. The amount of water vapor produced in the freeze dryer chamber 102 may decrease as the freeze-drying system 100 approaches the endpoint of the primary stage. In one embodiment, the freeze-drying system 100 reaches the endpoint of the primary drying stage when all of the free ice crystals in the product have been sublimated. In another embodiment, the freeze-drying system 100 reaches the endpoint of the primary drying stage when the amount of water vapor or the vapor pressure in the freeze dryer chamber 102 is below a threshold. At the end of the primary drying stage, moisture content may still be in the 5-10% range due to the presence of "sorbed" water molecules attached to the product (e.g., water molecules attached to the product by sorption).

During the secondary drying stage, the water molecules that are bound to the product are removed (desorbed). Since the free ice has been removed, the product temperature can be increased considerably without the risk of melting or collapse. Secondary drying continues until the product has an amount of moisture content that is acceptable for long term storage (e.g., 0.5-3%).

At block 720, processing logic receives a plurality of pressure measurements from a gauge during the primary drying stage. In one embodiment, the plurality of pressure measurements may include pressure measurements 402 from gauge 128 and pressure measurements 404 from capacitance manometer 300 as described in the description of FIG. 4. In one embodiment, the first valve 124 and liquid isolation valve 130 are in an open position during the primary drying stage. In another embodiment, the first valve 124 and liquid isolation valve 130 are in an open position during a portion of the primary drying stage.

At block 730, processing logic determines an endpoint of the primary drying stage from at least one of the plurality of pressure measurements. In one embodiment, the processing logic determines the endpoint of the primary drying stage when the pressure measurements 402 and the pressure measurements 404 converge as described in the description of FIG. 4.

At block 740, processing logic initiates the secondary drying stage subsequent to the determined endpoint. By initiating the secondary drying stage immediately or soon after the endpoint of the primary drying stage, the freeze-drying system 100 can avoid wasting time and energy by not remaining in the primary drying stage beyond the endpoint and the freeze-drying system 100 can avoid collapsing and damaging the product by not starting the secondary drying stage before the primary drying stage has completed.

It should be noted that the above described operations are just one method for using a freeze-drying system 100 and that, in alternative embodiments, certain ones of the operations of **FIG. 7** may be optional or take a simpler form.

It will be apparent from the foregoing description that aspects of the present disclosure may be embodied, at least in part, in software. That is, the techniques may be carried out in a computer system or other data processing system in response to a processing device, for example, executing sequences of instructions contained in a memory. In various embodiments, hardware circuitry may be used in combination with software instructions to implement the present disclosure. Thus, the techniques are not limited to any specific combination of hardware circuitry and software or to any particular source for the instructions executed by the data processing system. In addition, throughout this description, various functions and operations may be described as being performed by or caused by software code to simplify description. However, those skilled in the art will recognize what is meant by such expressions is that the functions result from execution of the code by processing device.

A machine-readable medium can be used to store software and data which when executed by a general purpose or special purpose data processing system causes the system to perform various methods of the present disclosure. This executable software and data may be stored in various places including, for example, system memory and storage or any other device that is capable of storing software programs and/or data. Thus, a machine-readable medium includes any mechanism that provides (i.e., stores) information in a form accessible by a machine (e.g., a computer, network device, personal digital assistant, manufacturing tool, any device with a set of one or more processors, etc.). For example, a machine-readable medium includes recordable/non-recordable media such as read only memory (ROM), random access memory (RAM), magnetic disk storage media, optical storage media, flash memory devices, etc.

Unless stated otherwise as apparent from the foregoing discussion, it will be appreciated that terms such as "maintaining," "receiving," "providing," "determining," "initiating," or the like may refer to the actions and processes of a computer system, or similar electronic computing device, that manipulates and transforms data represented as physical (e.g., electronic) quantities within the computer system's registers and memories into other data similarly represented as physical within the computer system memories or registers or other such information storage or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, embodiments of the present disclosure are not described with reference to any particular programming language. It will be appreciated that a variety of programming languages may be used to implement embodiments of the present disclosure.

In the foregoing description, numerous specific details are set forth, such as specific materials, dimensions, processes parameters, etc., to provide a thorough understanding of the embodiments of the present disclosure. The particular features, structures, materials, or characteristics may be combined in any suitable manner in one or more embodiments. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. In addition, the articles "a" and "an" as used in this application and the appended claims should generally be construed to mean "one or more" unless specified otherwise or clear from context to be directed to a singular form.

Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment, and such references mean "at least one".

It is to be understood that the above description is intended to be illustrative, and not restrictive. Many other embodiments will be apparent to those of skill in the art upon reading and understanding the above description. The scope of the disclosure should, therefore, be determined with reference to the appended claims, along with the full scope of equivalents to which such claims are entitled.

## Claims

1. An assembly (120) comprising:
a steam sterilizable filter (122) disposed downstream from a freeze dryer chamber (102);
a first valve (124) disposed downstream from the filter (122), wherein the first valve (124) is in a closed position during a steam sterilization cycle (SIP);
a second valve (126) disposed downstream from the steam sterilizable filter (122), wherein the second valve (126) is in an open position during the steam sterilization cycle (SIP); and
a gauge (128) disposed downstream from the first valve (124), wherein the gauge (128) is to determine a plurality of measurements of a freeze-drying system (100) associated with the freeze dryer chamber (102), wherein the first valve (124) in the closed position is configured to protect the gauge (128),
a main drain (116) downstream the second valve (126).

2. The assembly (120) of claim 1, wherein the gauge (128) is isolated from the freeze dryer chamber (102) by the first valve (124) during the steam sterilization cycle (SIP).

3. The assembly (120) of claim 1, wherein the gauge (128) comprises a thermocouple gauge.

4. The assembly of claim 1, wherein the gauge comprises a Pirani gauge.

5. The assembly (120) of claim 1, wherein the gauge (128) is to determine a first portion of the plurality of measurements before the steam sterilization cycle (SIP) and a second portion of the plurality of measurements after the steam sterilization cycle (SIP).

6. The assembly (120) of claim 1, wherein the assembly (120) is coupled to a processing device to determine, in view of at least one of the plurality of measurements, an endpoint of a primary drying stage of the freeze-drying system (100).

7. A method comprising:
maintaining a first valve (124) of an assembly (120) in a closed position during a steam sterilization cycle (SIP) of a freeze dryer chamber (102), wherein the assembly (120) is coupled to the freeze dryer chamber (102), the assembly (120) is configured according to any one of the previous claims 1 to 6;
maintaining the second valve (126) in an open position during the steam sterilization cycle (SIP) thus allowing steam entering the steam sterilizable filter (122) from the freeze dryer chamber (102), flowing through the steam sterilizable filter (122), exiting via the second valve (126) and continuing to a connection point to the main drain (116);
receiving, from the gauge (128), a plurality of pressure measurements of a freeze-drying system (100) associated with the freeze dryer chamber (102); and
determining, by a processing device in view of at least one of the plurality of pressure measurements, an endpoint of a primary drying stage of the freeze-drying system (100).

8. The method of claim 7 further comprises receiving, from the gauge (128), a second plurality of pressure measurements of the freeze-drying system (100) after the steam sterilization cycle (SIP), wherein the plurality of measurements are received, from the gauge (128), before the steam sterilization cycle (SIP).

9. The method of claim 7, wherein the gauge (128) is isolated from the freeze dryer chamber (102) by the first valve (SIP) during the steam sterilization cycle (SIP).

10. The method of claim 7 further comprising maintaining the first valve (124) in the open position during the primary drying stage.

11. The method of claim 7 further comprising initiating a secondary drying stage subsequent to the determined endpoint, wherein the at least one of the plurality of pressure measurements is received from the gauge (128) during the primary drying stage.

12. A freeze-drying system (100) comprising:
a freeze dryer chamber (102) comprising one or more orifices in fluid communication with the freeze dryer chamber (102); and
an assembly (120) according to any one of the previous claims 1 to 6 coupled to the freeze dryer chamber (102) via a first orifice of the one or more orifices.

13. The freeze-drying system of claim 12, wherein the first orifice comprises a chamber port (110), and wherein the one or more orifices further comprise:
a second orifice comprising a steam inlet (112), wherein the steam inlet (112) is disposed upstream from the chamber port (110); and
a third orifice comprising a chamber drain orifice (114), wherein the chamber drain orifice (114) is disposed downstream from the steam inlet and upstream from the main drain (116).

14. The freeze-drying system of claim 12 further comprising a chamber drain isolation valve (118) disposed downstream from the chamber drain orifice (114), downstream from the second valve (126), and upstream from the main drain (116).

15. The freeze-drying system of claim 12 further comprising a processing device to determine, in view of at least one of the plurality of measurements, an endpoint of a primary drying stage.

## Patentansprüche

1. Anordnung (120) umfassend:
einen dampfsterilisierbaren Filter (122), der stromabwärts von einer Gefriertrocknerkammer (102) angeordnet ist;
ein erstes Ventil (124), das stromabwärts von dem Filter (122) angeordnet ist, wobei das erste Ventil (124) sich während eines Dampfsterilisationszyklus (SIP) in einer geschlossenen Position befindet;
ein zweites Ventil (126), das stromabwärts des dampfsterilisierbaren Filters (122) angeordnet ist,
wobei sich das zweite Ventil (126) während des Dampfsterilisationszyklus (SIP) in einer offenen Stellung befindet;
und
ein Messgerät (128), das stromabwärts von dem ersten Ventil (124) angeordnet ist, wobei das Messgerät (128) dazu dient, eine Vielzahl von Messungen eines Gefriertrocknungssystems (100) zu bestimmen, das mit der Gefriertrocknerkammer (102) verbunden ist, wobei das erste Ventil (124) in der geschlossenen Position so konfiguriert ist, dass es das Messgerät (128) schützt,
einen Hauptablass (116) stromabwärts des zweiten Ventils (126).

2. Anordnung (120) nach Anspruch 1, bei der das Messgerät (128) während des Dampfsterilisationszyklus (SIP) durch das erste Ventil (124) von der Gefriertrocknerkammer (102) isoliert ist.

3. Anordnung (120) nach Anspruch 1, wobei das Messgerät (128) ein Thermoelement-Messgerät umfasst.

4. Die Baugruppe aus Anspruch 1, wobei das Messgerät ein Pirani-Messgerät umfasst.

5. Anordnung (120) nach Anspruch 1, wobei das Messgerät (128) einen ersten Teil der Mehrzahl von Messungen vor dem Dampfsterilisationszyklus (SIP) und einen zweiten Teil der Mehrzahl von Messungen nach dem Dampfsterilisationszyklus (SIP) bestimmen soll.

6. Anordnung (120) nach Anspruch 1, wobei. die Anordnung (120) mit einer Verarbeitungsvorrichtung gekoppelt ist, um im Hinblick auf mindestens eine der Vielzahl von Messungen einen Endpunkt einer primären Trocknungsstufe des Gefriertrocknungssystems (100) zu bestimmen.

7. Verfahren umfassend:
Halten eines ersten Ventils (124) einer Anordnung (120) in einer geschlossenen Position während eines Dampfsterilisationszyklus (SIP) einer Gefriertrocknungskammer (102), wobei die Anordnung (120) mit der Gefriertrocknungskammer (102) gekoppelt ist, die Anordnung (120) gemäß einem der vorhergehenden Ansprüche 1 bis 6 konfiguriert ist;
Halten des zweiten Ventils (126) in einer offenen Stellung während des Dampfsterilisationszyklus (SIP), wodurch Dampf in den dampfsterilisierbaren Filter (122) aus der Gefriertrocknungskammer (102) eintritt, durch den dampfsterilisierbaren Filter (122) strömt, über das zweite Ventil (126) austritt und zu einem Verbindungspunkt zum Hauptablass (116) weitergeht;
Empfangen einer Vielzahl von Druckmessungen eines Gefriertrocknungssystems (100), das mit der Gefriertrocknungskammer (102) verbunden ist, von dem Messgerät (128); und
Bestimmung eines Endpunktes einer primären Trocknungsstufe des Gefriertrocknungssystems (100) durch eine Verarbeitungsvorrichtung im Hinblick auf mindestens eine der mehreren Druckmessungen.

8. Verfahren nach Anspruch 7 ferner umfassend das Empfangen einer zweiten Vielzahl von Druckmessungen des Gefriertrocknungssystems (100) von dem Messgerät (128) nach dem Dampfsterilisationszyklus (SIP), wobei die Vielzahl von Messungen von dem Messgerät (128) vor dem Dampfsterilisationszyklus (SIP) empfangen wird.

9. Verfahren nach Anspruch 7, wobei das Messgerät (128) während des Dampfsterilisationszyklus (SIP) durch das erste Ventil (SIP) von der Gefriertrocknungskammer (102) isoliert ist.

10. Verfahren nach Anspruch 7 ferner umfassend das Halten des ersten Ventils (124) in der offenen Stellung während der primären Trocknungsstufe.

11. Verfahren nach Anspruch 7, das weiterhin das Initiieren einer sekundären Trocknungsstufe im Anschluss an den bestimmten Endpunkt umfasst, wobei die mindestens eine der mehreren Druckmessungen von dem Messgerät (128) während der primären Trocknungsstufe empfangen wird.

12. Gefriertrocknungssystem (100) umfassend:
eine Gefriertrocknungskammer (102), die eine oder mehrere Öffnungen in Fluidverbindung mit der Gefriertrocknungskammer (102) aufweist; und eine Anordnung (120) nach einem der vorstehenden Ansprüche 1 bis 6, die mit der Gefriertrocknungskammer (102) über eine erste Öffnung der einen oder mehreren Öffnungen verbunden ist.

13. Gefriertrocknungssystem nach Anspruch 12, wobei die erste Öffnung eine Kammeröffnung (110) umfasst; und wobei die eine oder mehrere Öffnungen weiterhin eine oder mehrere Öffnungen umfassen:
eine zweite Öffnung mit einem Dampfeinlass (112), wobei der Dampfeinlass (112) stromaufwärts von der Kammeröffnung (110) angeordnet ist; und
eine dritte Öffnung, die eine Kammerablassöffnung (114) umfasst, wobei die Kammerablassöffnung (114) stromabwärts von dem Dampfeinlass und stromaufwärts von dem Hauptablass (116) angeordnet ist.

14. Gefriertrocknungssystem nach Anspruch 12 ferner umfassend ein Kammerabfluss-Absperrventil (118), das stromabwärts von der Kammerabflussöffnung (114), stromabwärts von dem zweiten Ventil (126) und stromaufwärts von dem Hauptabfluss (116) angeordnet ist.

15. Gefriertrocknungssystem nach Anspruch 12 ferner umfassend eine Verarbeitungsvorrichtung, um im Hinblick auf mindestens eine der Vielzahl von Messungen einen Endpunkt einer primären Trocknungsstufe zu bestimmen.

## Revendications

1. Ensemble (120) comprenant :
un filtre stérilisable à la vapeur (122) disposé en aval d'une chambre de lyophilisation (102) ;
une première vanne (124) disposée en aval du filtre (122), la première vanne (124) étant dans une position fermée pendant un cycle de stérilisation à la vapeur (SIP) ;
une seconde vanne (126) disposée en aval du filtre stérilisable à la vapeur (122), la seconde vanne (126) étant dans une position ouverte pendant le cycle de stérilisation à la vapeur (SIP) ; et
une jauge (128) disposée en aval de la première vanne (124), la jauge (128) étant destinée à déterminer une pluralité de mesures d'un système de lyophilisation (100) associé à la chambre de lyophilisation (102), la première vanne (124) en position fermée étant conçue pour protéger la jauge (128),
un drain principal (116) en aval de la seconde vanne (126).

2. Ensemble (120) selon la revendication 1, dans lequel la jauge (128) est isolée de la chambre de lyophilisation (102) par la première vanne (124) pendant le cycle de stérilisation à la vapeur (SIP).

3. Ensemble (120) selon la revendication 1, dans lequel la jauge (128) comprend une jauge à thermocouple.

4. Ensemble selon la revendication 1, dans lequel la jauge comprend une jauge Pirani.

5. Ensemble (120) selon la revendication 1, dans lequel la jauge (128) est destinée à déterminer une première partie de la pluralité de mesures avant le cycle de stérilisation à la vapeur (SIP) et une deuxième partie de la pluralité de mesures après le cycle de stérilisation à la vapeur (SIP).

6. Ensemble (120) selon la revendication 1, où l'ensemble (120) est couplé à un dispositif de traitement pour déterminer, en tenant compte d'au moins l'une de la pluralité de mesures, un point limite d'une étape de séchage primaire du système de lyophilisation (100).

7. Procédé comprenant :
le maintien d'une première vanne (124) d'un ensemble (120) dans une position fermée pendant un cycle de stérilisation à la vapeur (SIP) d'une chambre de lyophilisation (102), l'ensemble (120) étant couplé à la chambre de lyophilisation (102), l'ensemble (120) étant conçu selon l'une quelconque des revendications précédentes 1 à 6 ;
le maintien de la seconde vanne (126) dans une position ouverte pendant le cycle de stérilisation à la vapeur (SIP) permettant ainsi à la vapeur de pénétrer dans le filtre stérilisable à la vapeur (122) depuis la chambre de lyophilisation (102), de s'écouler à travers le filtre stérilisable à la vapeur (122), de sortir via la seconde vanne (126) et de continuer jusqu'à un point de connexion au drain principal (116) ;
la réception, depuis la jauge (128), d'une pluralité de mesures de pression d'un système de lyophilisation (100) associé à la chambre de lyophilisation (102) ; et
la détermination, par un dispositif de traitement en tenant compte d'au moins l'une de la pluralité de mesures de pression, d'un point limite d'une étape de séchage primaire du système de lyophilisation (100).

8. Procédé selon la revendication 7, comprenant en outre la réception, depuis la jauge (128), d'une deuxième pluralité de mesures de pression du système de lyophilisation (100) après le cycle de stérilisation à la vapeur (SIP), où la pluralité de mesures est reçue, depuis la jauge (128), avant le cycle de stérilisation à la vapeur (SIP).

9. Procédé selon la revendication 7, où la jauge (128) est isolée de la chambre de lyophilisation (102) par la première vanne (SIP) pendant le cycle de stérilisation à la vapeur (SIP).

10. Procédé selon la revendication 7, comprenant en outre le maintien de la première vanne (124) en position ouverte pendant l'étape de séchage primaire.

11. Procédé selon la revendication 7, comprenant en outre le lancement d'une étape de séchage secondaire postérieurement au point limite déterminé, l'au moins une de la pluralité de mesures de pression étant reçue depuis la jauge (128) pendant l'étape de séchage primaire.

12. Système de lyophilisation (100) comprenant :
une chambre de lyophilisation (102) comprenant un ou plusieurs orifices en communication fluidique avec la chambre de lyophilisation (102) ; et
un ensemble (120) selon l'une quelconque des revendications 1 à 6 précédentes couplé à la chambre de lyophilisation (102) via un premier orifice parmi le ou les orifices.

13. Système de lyophilisation selon la revendication 12, dans lequel le premier orifice comprend un orifice de chambre (110), et où le ou les orifices comprennent en outre :
un deuxième orifice comprenant une entrée de vapeur (112), l'entrée de vapeur (112) étant disposée en amont de l'orifice de la chambre (110) ; et
un troisième orifice comprenant un orifice de vidange de chambre (114), l'orifice de vidange de chambre (114) étant disposé en aval de l'entrée de vapeur et en amont du drain principal (116).

14. Système de lyophilisation selon la revendication 12, comprenant en outre une vanne d'isolation de vidange de chambre (118) disposée en aval de l'orifice de vidange de chambre (114), en aval de la seconde vanne (126) et en amont du drain principal (116).

15. Système de lyophilisation selon la revendication 12, comprenant en outre un dispositif de traitement pour déterminer, en tenant compte d'au moins l'une de la pluralité de mesures, un point limite d'une étape de séchage primaire.
